# EUROPEAN PATENT SPECIFICATION

(11) **EP 0 959 843 B1**
(45) Date of publication and mention of the grant of the patent: **16.10.2002**
(21) Application number: 97922543.0
(22) Date of filing: 01.05.1997
(51) Int. Cl.: A61F 13/15

(54) **ABSORBENT ARTICLE HAVING INTERNAL SIDE WRAPPING ELEMENTS**
ABSORBIERENDER ARTIKEL MIT INTERNEN SEITENUMHÜLLUNGSELEMENTEN
ARTICLE ABSORBANT COMPORTANT DES ELEMENTS LATERAUX ET INTERNES ENVELOPPANTS

(30) Priority: 08.05.1996 US 646459
(43) Date of publication of application: 01.12.1999
(73) Proprietor: THE PROCTER & GAMBLE COMPANY, Cincinnati, Ohio 45202 (US)
(72) Inventor: HINES, Letha, Margie, Cincinnati, OH 45215 (US); BROWN, Robert, Alan, Maineville, OH 45039 (US); SNAUWAERT, Sofie, Gilberte, Cincinnati, OH 45227 (US); FERESHTEHKHOU, Saeed, Fairfield, OH 45014 (US); HAMMONS, John, Lee, Hamilton, OH 45011 (US)
(74) Representative: Kremer, Véronique
(86) International application number: US9707203
(87) International publication number: WO97041818

(56) References cited:
- EP-A- 0 467 184
- EP-A- 0 670 153
- EP-A- 0 692 232
- WO-A-93/01781
- WO-A-93/01785
- WO-A-95/15139
- WO-A-95/28137

## Description

### FIELD OF THE INVENTION

The present invention relates to absorbent articles such as sanitary napkins, panty liners, and adult incontinence pads. More particularly, the present invention relates to absorbent articles of the foregoing type which provide improved coverage of the wearer's undergarments, and which have side wrapping elements that fold around or wrap the sides of a wearer's undergarments, and relates to a method of making such an absorbent article such as sanitary napkin.

### BACKGROUND OF THE INVENTION

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perineal area of the body.

All manner and variety of absorbent articles configured for the absorption of body fluids such as menses, urine, and feces are, of course, well known. Absorbent articles, particularly sanitary napkins, having wings or flaps are disclosed in the literature and are available in the marketplace.

Generally, the flaps extend laterally from a central absorbent means and are intended to be folded around the edges of the wearer's panties in the crotch region. Commonly, the flaps are provided with an attachment means for affixing the flaps to the underside of the wearer's panties or to the opposing flap. The flaps are generally effective, to varying degrees in preventing exudates from soiling the edges of the wearer's panties.

Sanitary napkins having flaps of various types are disclosed in U.S. Patent 4,687,478, entitled "Shaped Sanitary Napkin With Flaps", which issued to Van Tilburg on August 18, 1987, U.S. Patent 4,608,047, entitled "Sanitary Napkin Attachment Means", which issued to Mattingly on August 26, 1986, U.S. Patent 4,589,876, entitled "Sanitary Napkin", which issued to Van Tilburg on May 20, 1986, U.S. Patent 4,285,343, entitled "Sanitary Napkin", which issued to McNair on August 25, 1981, U.S. Patent 3,397,697, entitled "Disposable Sanitary Shield For Undergarments", which issued to Rickard on August 20, 1968, and U.S. Patent 2,787,271, entitled "Sanitary Napkin", which issued to Clark on April 2, 1957.

While sanitary napkins having flaps are commonly viewed as providing better protection against soiling as compared to sanitary napkins without flaps, the flapped napkins commonly experience problems that keep them from being optimally effective. These problems generally result from the stresses exerted on the flaps when the sanitary napkins are worn.

When the flaps are folded down along the edges of the wearer's panties, stresses are created in the flaps, particularly when the flaps are relatively large in size. The stresses are especially high along the fold line at the edges of the wearer's panties where the flaps are bent from the body side of the panty to the underside of the panty. These stresses are caused by fitting a flap around the curved edges of a panty crotch. The stresses are magnified when a wearer sits or crouches because the edges of the panties are pulled outward against the flaps thus increasing the forces against this fold line. When the stresses become too high, the flaps may become detached from the panty and some portion of the aforementioned benefits of the flaps may be lost. In addition, even if the stresses are not sufficient to detach the flaps, they may still be sufficient to cause the flaps to bunch longitudinally inward. This effectively reduces the size of the flaps and the area of the wearer's undergarments that the flaps are able to cover. Thus, there is a commercial need for a way of eliminating or at least reducing the stresses that develop in the flaps when folded, so as to prevent them from becoming detached from the wearer's panties and losing ability to cover a given area of the panties.

A number of variations on the types of flaps described above have been presented in an attempt to solve these problems. A sanitary napkin having flaps with stress relief means in the form of a notch or a slit is described in U.S. Patent 4,917,697 which issued to Osborn, III, et al. on April 17, 1990. Absorbent articles having flaps and zones of differential extensibility for relieving the stresses which develop in the flaps are disclosed in U.S. Patent 5,344,416 issued to Niihara on September 6, 1994, and U.S. Patent 5,354,400 issued to Lavash, et al. on October 11, 1994. Sanitary napkins with different alternative types of flaps are also described in EP 467184, WO 93/01871, and WO 95/28137. A method for making absorbent structures, for example for the production of sanitary napkins, is described in EP 670153. Although these sanitary napkins work quite well, the search for sanitary napkins having improved flaps has continued.

Therefore, it is an object of the present invention to provide an absorbent article, such as a sanitary napkin, having flaps that provides the absorbent article with further improved means for relieving the stresses that develop in the flaps when they are folded down along the edges of the crotch of the wearer's undergarments and affixed to the underside of the undergarments. It is another object of the present invention to provide such an absorbent article with flaps that do not lose their ability to cover a given area of the wearer's undergarments during wear.

These and other objects of the present invention wilt be more readily apparent when considered in reference to the following description and when taken in conjunction with the accompanying drawings.

### SUMMARY OF THE PRESENT INVENTION

The present invention is directed to absorbent articles such as sanitary napkins, panty liners, and adult incontinence pads which provide improved coverage of the wearer's undergarments to reduce soiling of the wearer's undergarments. The absorbent articles have a particular type of side wrapping elements that fold or wrap the sides of a wearer's undergarments.

The absorbent article has a principal longitudinal centerline and a principal transverse centerline, and comprises a main body portion having two spaced apart longitudinal edges and two spaced apart transverse edges. The main body portion comprises a liquid pervious topsheet, a liquid impervious backsheet joined to the topsheet, and an absorbent core positioned between the topsheet and the backsheet. The main body portion has a first end region, a second end region, and a central region disposed between the first and second end regions. The first and second end regions preferably comprise lobes that project laterally outward from in each longitudinal edge so that the main body portion is narrower in width measured across its central region than at its end regions. A portion of the longitudinal side edges in the region of the lobes, thus, defines the laterally outwardmost portion of the main body portion.

The absorbent article further comprises a pair of side extensions, or side wrapping elements for folding around the side edges of the wearer's undergarment. The side wrapping elements have a proximal edge, a distal edge, and a side wrapping element transverse centerline that intersects the principal longitudinal centerline of the absorbent article. The side wrapping elements are joined to the main body portion at their proximal edges and extend laterally outward to their distal edges (or "free ends") from at least said central region of said main body portion. The side extensions have a surface area wherein the majority of the surface area of the side extensions is located laterally inward of the laterally outwardmost portion of said main body portion.

There are numerous advantages that are provided by the absorbent article of the present invention in use, and during manufacture. The side wrapping elements are more flexible than the adjacent curvilinear longitudinal side edges of the main body portion of the sanitary napkin. The side wrapping elements are also provided with weakened regions that allow the side wrapping elements to fold smoothly around the edges of the wearer's undergarment. If the longitudinal side edges of the main body portion are approximately the same size and shape as the side edges of the wearer's undergarment, the side wrapping elements can fold on a curvilinear line virtually exactly along the side edges of the wearer's undergarments. The fact that the side wrapping elements can fold along a curvilinear line, allows the side wrapping elements to form a flat fold along the length of the edges of the wearer's panties. This substantially reduces, if not eliminates, any tendency for the side wrapping elements to become unattached to the underside of the wearer's panties (or for the fastener on the side wrapping elements to "pop" off from their attachment with the underside of the panties).

Several novel releasable wrapper configurations are also provided for enclosing and protecting the sanitary napkin prior to use. In one embodiment, the lobes of the sanitary napkin are folded inward over the topsheet to expose adhesive patches on the lobes and the side wrapping elements. The exposed adhesive patches are covered with a sheet of release paper. The fastener on the garment facing side of the sanitary napkin is placed on a releasable wrapper, and the sanitary napkin and releasable wrapper are folded about a pair of transverse axes to form an individual package for the sanitary napkin. This embodiment provides the advantage that it produces a relatively small and convenient package for a relatively large pad.

In another embodiment, the adhesive fasteners on the garment facing side of the main body portion can be covered with multiple release papers for ease of removal. These multiple release papers can comprise separate pieces that have overlapping portions, or they can comprise an individual release paper that is perforated so that it can be separated into more than one piece for removal.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:
Fig. 1 is a top plan view of a preferred embodiment of a sanitary napkin of the present invention.
Fig. 2 is a cross-sectional view of the sanitary napkin shown in Fig. 1, taken along line 2-2.
Fig. 3 is a bottom plan view of the sanitary napkin shown in Fig. 1 which shows one possible panty fastener pattern.
Fig. 4 is an exploded perspective view showing the assembly of the components of the sanitary napkin shown in Fig. 1.
Fig. 4A is a top plan view of the secondary absorbent structure of the sanitary napkin shown in the preceding drawing figures with cuts formed therein.
Fig. 4B is a top plan view of the secondary absorbent structure in Fig. 4A, showing how the cut portions thereof may be folded inward to form recessed areas for the side wrapping elements of the sanitary napkin.
Fig. 5 is a bottom plan view of the sanitary napkin shown in Fig. 1 which shows another possible panty fastener pattern.
Fig. 6 is a top plan view of the sanitary napkin shown in Fig. 5 when it is folded and placed on a releasable wrapper that will serve as an individual package for the sanitary napkin.
Fig. 7 is a side view of the sanitary napkin and releasable wrapper shown in Fig. 6 in a partially folded configuration.
Fig. 8 is a perspective view of the sanitary napkin of Fig. 7 with the releasable wrapper completely folded around the sanitary napkin to form an individual package for the sanitary napkin.
Fig. 9 is a perspective view of the sanitary napkin of the present invention in a wearer's panties.
Fig. 10 is a top plan view of the sanitary napkin of the present invention which is provided with a multiple piece panty fastener cover.
Fig. 11 is a bottom plan view of the sanitary napkin shown in Fig. 10.
Fig. 12 is a bottom plan view of a sanitary napkin having a perforated panty fastener cover that can be separated into more than one piece for removal.

### DETAILED DESCRIPTION OF THE INVENTION

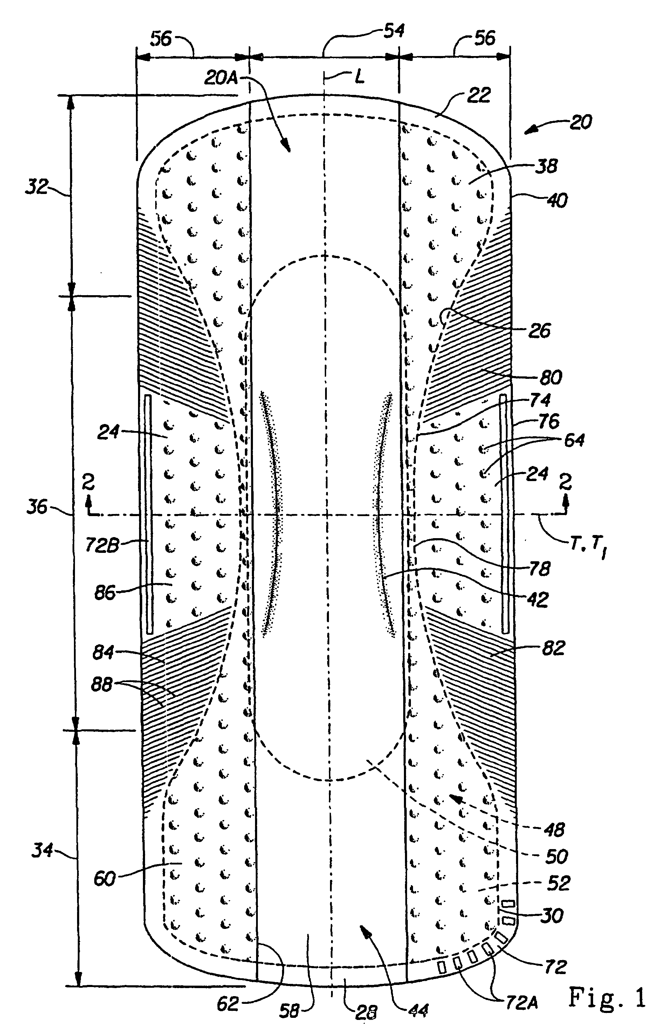

The present invention relates to absorbent articles, such as sanitary napkins, panty liners, and incontinence pads. FIGS. 1-3 show one preferred embodiment of a disposable absorbent article of the present invention, sanitary napkin 20. As shown in Figure 1, the sanitary napkin 20 basically comprises a main body portion 22 and two side extensions or side wrapping elements 24.

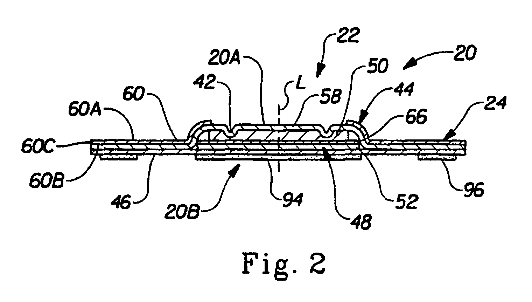

The sanitary napkin 20 (and the main body portion thereof) has two surfaces, a liquid pervious body-contacting surface or "body surface" 20A and a liquid impervious garment surface 20B. The sanitary napkin 20 is shown in FIG. 1 as viewed from its body surface 20A. The body surface 20A is intended to be worn adjacent to the body of the wearer. The garment surface 20B of the sanitary napkin 20 (shown in FIG. 2) is on the opposite side and is intended to be placed adjacent to the wearer's undergarments when the sanitary napkin 20 is worn.

The sanitary napkin 20 has two centerlines, a principal longitudinal centerline L and a principal transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

FIG. 1 shows that the main body portion 22 of the sanitary napkin 20 comprises the portion of the sanitary napkin without the side wrapping elements 24. The main body portion 22 has two spaced apart longitudinal edges 26, two spaced apart transverse or end edges (or "ends") 28, which together form the periphery 30 of the main body portion of the sanitary napkin 20. The main body portion 22 also has two end regions, which are designated first end region 32 and second end region 34. A central region 36 is disposed between the end regions 32 and 34. The end regions 32 and 34 extend outwardly from the edges of the central region 36 about 1/8 to about 1/3 of the length of the main body portion. A detailed description of the central region and two end regions for a sanitary napkin is contained in U.S. Patent 4,690,680 issued to Higgins on September 1, 1987.

The main body portion 22 of the sanitary napkin 20 is preferably hourglass shaped or dog bone shaped. The first and second end regions 32 and 34, of the main body portion 22 preferably comprise lobes 38 that extend laterally outward at each longitudinal edge 26 of the main body portion so that the main body portion 22 is narrower in width when measured across the central region 36 than at its end regions 32 and 34. The outermost edges of the lobes 38, thus define portions of the longitudinal side edges 26 of the main body portion 22. A portion of the longitudinal side edges 26 in the region of the lobes 38 will typically define a laterally outwardmost portion 40 of the main body portion 22.

The main body portion 22 of the sanitary napkin 20 can be of any thickness, including relatively thick, intermediate (or moderate) thickness, relatively thin, or even very thin. The embodiment of the sanitary napkin 20 shown in Figures 1-3 of the drawings is intended to be an example of a moderately thick sanitary napkin. In this embodiment, however, the main body portion 22 is generally thinner in the end regions 32 and 34 than in the central region 36 so that it will be more comfortable and discrete than if it was uniformly moderately thick. The main body portion 22 of the sanitary napkin is also preferably embossed with channels 42 such as those described in U.S. Patents 5,234,422 and 5,308,346 issued to Snelier, et al. It should be understood that the sanitary napkin shown is merely one preferred embodiment, and that the present invention is not limited to absorbent articles of the type or having the specific configurations shown in the drawings.

The sanitary napkin 20 shown in FIG. 1 can be of any suitable size. Preferably, the sanitary napkin 20 is a size sufficient to allow the side wrapping elements 24 to fold as described in greater detail herein. The sanitary napkin 20, and the main body portion 22 thereof, are preferably also relatively large in size so that they are able to cover the maximum area of the wearer's panties to reduce or eliminate soiling of the same by the wearer's bodily fluids. In one preferred embodiment, the main body portion 22 of the sanitary napkin 20 is about 3.25 inches (8.26 cm) wide at its narrowest point. The overall sanitary napkin 20 in such an embodiment is approximately 14.75 inches (37.5 cm) in length measured along the longitudinal centerline L, and about 6.25 inches (about 16 cm) in width (measured between the distal edges of the side wrapping elements). In another embodiment, the width of the sanitary napkin 20 is the same, but the length ranges from about 31.7 cm to about 34.5 cm.

FIG. 2 shows the individual components of the main body portion 22 of the sanitary napkin 20 of the present invention. The main body portion 22 generally comprises at least three primary components. These include a liquid pervious topsheet 44, a liquid impervious backsheet 46, and an overall absorbent portion (or "absorbent core") 48 positioned between the topsheet 44 and the backsheet 46. The absorbent core 48 preferably comprises a main (or primary) absorbent structure (or absorbent component) 50 and a secondary absorbent structure (or absorbent component) 52. The main absorbent component 50 is preferably roughly centered along the principal longitudinal and transverse centerlines L and T. In alternative embodiments, the main absorbent component 50 could be shifted longitudinally forward or backward relative to the transverse centerline T. Suitable materials for the various components of the sanitary napkin 20 shown in FIG. 2 are described below.

In a particularly preferred embodiment, the topsheet 44 comprises a composite or "hybrid" topsheet structure. The hybrid topsheet 44 generally comprises a longitudinally oriented central zone 54 and longitudinal side regions 56 located laterally outboard of the central zone 54. The hybrid topsheet 44 structure preferably comprises an apertured thermoplastic film 58 in the central zone 54 of the hybrid topsheet structure 44 and a less plastic-like outer covering that forms the longitudinal side regions 56 of the topsheet. Such a topsheet 44 is useful for improving the skin feel and comfort of topsheets made of apertured plastic films. In particular, such a topsheet 44 reduces the tendency for apertured plastic films to feel hot, sweaty, and sticky. This is especially useful in the case of relatively large absorbent articles such as the one shown in FIGS. 1-3 where there is a large portion of the topsheet 44 which is in contact with the wearer's body. Hybrid topsheet structures (though not the particularly preferred hybrid topsheet used herein) are described generally in PCT Publication No. 93/09744 assigned to The Procter & Gamble Company which published May 27, 1993, in the name of Sugahara.

In the particularly preferred embodiment shown in FIGS. 1-3, the apertured thermoplastic film 58 of the hybrid topsheet 44 preferably extends the full width of the sanitary napkin 20. The apertured thermoplastic film 58 preferably comprises an apertured film sold on sanitary napkins by The Procter & Gamble Company of Cincinnati, Ohio under the trademark DRI-WEAVE, which is manufactured under U.S. Patent 4,342,314 issued to Radel, et al. on August 3, 1982 and U.S. Patent 4,463,045 issued to Ahr, et al. on July 31, 1984. In preferred embodiments, the apertured thermoplastic film 58 is rendered hydrophilic so that liquids will transfer through the apertured thermoplastic film faster. This will diminish the likelihood that body exudates will flow off the apertured thermoplastic film 58 rather than being drawn through the apertured thermoplastic film and being absorbed by the absorbent core 48. The apertured thermoplastic film 58 can be rendered hydrophilic by treating it with surfactants. Suitable methods of applying surfactants are described in U.S. Patents 4,950,254 and 5,009,653 issued to Osborn. Preferably, surfactant is incorporated into the resin used to make the apertured film.

The less plastic-like outer covering 60 can be any suitable type of material (or web of material) that is liquid pervious and is more comfortable to the wearer's skin than the apertured film 58 that forms the central zone 54 of the topsheet. Suitable materials include less plastic-like apertured films and nonwoven materials. Suitable less plastic-like apertured films include the apertured film described in U.S. Patent 4,629,643 entitled "Microapertured Polymeric Web Exhibiting Soft and Silky Tactile Impression" issued to Curro, et al. on December 16, 1986, and the hydroformed films made by the method described in U.S. Patent 4,695,422 entitled "Production of Formed Material by Solid-State Formation With High-Pressure Liquid Stream" issued to Curro, et al. on September 22, 1987. Suitable nonwoven materials can be made from natural fibers (e.g., wood or cotton fibers), synthetic fibers (e.g., polyester, polyethylene, or polypropylene fibers), or a combination of natural and synthetic fibers. Such a nonwoven outer covering can be made by a number of manufacturing techniques. For example, a nonwoven outer covering can be spunbonded, carded, wet-laid, meltblown, hydroentangled, to name a few possible types of processes.

In the embodiment shown in FIGS. 1-3, the outer covering 60 is a nonwoven material (or nonwoven web). The nonwoven material 60, as shown in FIG. 2, has a body surface 60A and a garment facing surface 60B that are separated from one another by an intermediate portion 60C. The nonwoven material 60 can comprise any suitable type of nonwoven material described above. Preferably, in the embodiment shown in FIGS. 1-3, the nonwoven material 60 comprises a 23 gsm spunbond polyethylene nonwoven material known as COROLIND available from Corovin GmbH of Peine, Germany.

The spunbond polyethylene nonwoven material is preferably treated so that the body surface 60A of the nonwoven material exhibits a surface energy that is less than the surface energy of the intermediate portion 60C of the nonwoven material. That is, there is a surface energy gradient between these portions of the nonwoven material. The term "surface energy" as used herein, refers to the energy required to separate a liquid from a solid surface (e.g., a film or, in this case, a fiber). In a particularly preferred embodiment, the surface of the treated nonwoven material also exhibits a plurality of regions of comparatively low surface energy which define surface energy gradients (or "SEG") where they interface with higher surface energy web surfaces. Webs having such surface energy gradients are described in greater detail in U.S. Patent Application Serial No. 08/442,935, filed on May 31, 1995 in the name of Ouellette, et al. (PCT Publication WO 96/00548 published January 11, 1996), the disclosure of which is incorporated herein by reference. The treatment of the web to form a surface energy gradient, may be referred to herein as an "SEG" treatment for brevity. The SEG treatment is intended to reduce the hydrophilicity of the nonwoven material 60 and improve the stain masking and rewet properties of the nonwoven material. The nonwoven material 60 will have a reduced tendency to hold liquids at its surface, and liquids, such as menses, which are deposited on the nonwoven material will be better able to pass through the nonwoven material 60 to the underlying absorbent components. As a result, the liquid handling properties and masking properties of the nonwoven material 60 will more closely approach those of the apertured film 58.

The SEG treatment can be applied either before or after the components of the hybrid topsheet are assembled. However, the SEG treatment is preferably applied before assembly of the components so that it can be applied more precisely. The SEG treatment is preferably accomplished by applying a suitable surface treatment to the nonwoven web 60. A suitable surface treatment is a silicone release coating from Dow Corning of Midland, Michigan known as SYL-OFF 7677 to which a crosslinker known as SYL-OFF 7048 is added in proportions by weight of 100 parts to 10 parts, respectively. Another suitable surface treatment is a coating of a UV curable silicone comprising a blend of two silicones commercially available from General Electric Company, Silicone Products Division of Waterford, NY, under the designations UV 9300 and UV 9380C-D1, in proportions by weight of 100 parts to 2.5 parts, respectively. The surface treatments may be applied to the body surface 60A of the nonwoven web by techniques known in the art such as screen printing, gravure printing, spraying, dip coating, etc.

The nonwoven material 60 is preferably coated with silicone on the wearer-contacting surface at a basis weight of 1 gram per square meter. The basis weight of the SYL-OFF material is determined by subtracting the basis weight of the nonwoven material in an uncoated condition (grams per square mater) from the basis weight (grams per square mater) of the coated nonwoven material.

In addition to the liquid handling characteristics supplied by SEG treating the nonwoven component 60 of the hybrid topsheet 44, the treatment has also been found to improve the tear and tensile strength of the nonwoven material 60. The SEG treatment is believed to reduce the modulus of elasticity of the fiber network in the nonwoven material, making it softer, more flexible, and more drapable. This is useful when subsequent mechanical operations, such as the ring rolling process described below, will be performed on this portion of the hybrid topsheet 44 in order to provide the more flexible and extensible regions 84 described below.

The nonwoven material 60 is preferably secured in at least partial contacting relation with the apertured thermoplastic film component 58 of the hybrid topsheet 44. The nonwoven material 60 can be maintained in contact with the apertured thermoplastic film 58 by fusion bonding, adhesive attachment of the layers, or by any other suitable securement means known in the art. Fusion bonding includes heat and/or pressure bonding, ultrasonic bonding, and the like. The two layers can be continuously, partially, or intermittently bonded together. The bonding of the nonwoven material 60 to the apertured thermoplastic film 58 can prevent the nonwoven material 60 from being torn or from rolling back onto itself at its inside edges 62, which would create an uncomfortable feeling. In a preferred embodiment, the nonwoven material 60 and the apertured thermoplastic film layer 58 are adhesively bonded together using a spiral pattern of adhesives and are also spot-bonded with a plurality of small spaced apart circular fusion bonds 64.

Various alternative embodiments of the hybrid topsheet 44 are possible. In alternative embodiments of the hybrid topsheet, the nonwoven material (or other less plastic-like outer covering) can be of a different color from the apertured film 58. This can be used to provide the product with the appearance of a barrier at the boundary of the apertured film 58 and the nonwoven material (or other less plastic-like outer covering) 60. Examples of suitable colors for the nonwoven material (or other less plastic-like outer covering) 60 would be pink, peach, light blue, or lavender. Preferably, however, the nonwoven material (or other less plastic-like outer covering) 60 is white.

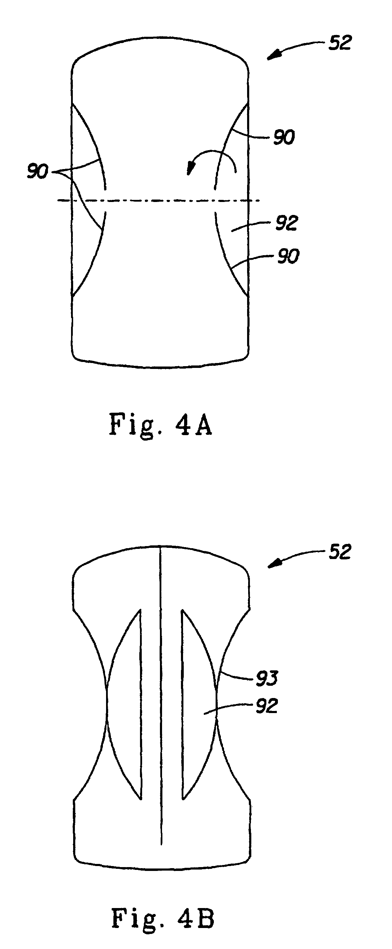

The absorbent core 48, as noted above, preferably comprises a primary absorbent structure 50. The primary absorbent structure 50 lies beneath the hybrid topsheet 44. The primary absorbent structure 50 serves as the main absorbent component of the sanitary napkin 20. The primary absorbent structure 50 absorbs the bodily exudates that are deposited directly on the sanitary napkin in the area of typical liquid deposition. The primary absorbent structure 50 can be any suitable type of absorbent structure. Suitable absorbent structures are described in the patents which are incorporated herein by reference. In the preferred embodiment, shown in FIGS. 1-4, the primary absorbent structure 50 comprises an oval or race-track shaped airfelt absorbent component similar to the absorbent core described in U.S. Patent U.S. Patents 5,234,422 and 5,308,346 issued to Sneller, et al. As shown in FIG. 4, the primary absorbent structure 50 is "profiled" so that it is provided with a central region which is thicker than its edges and ends. The primary absorbent structure 50 can be oriented so that the profiled central region is facing upward as shown in FIG. 4, or downward.

The secondary absorbent structure 52 serves as back-up absorbent for the primary absorbent structure 50. The secondary absorbent structure 52 is intended to absorb and contain bodily exudates that are not deposited directly on the primary absorbent structure 50, or that flow across the topsheet beyond the boundaries of the primary absorbent structure, or which overload any portions of the primary absorbent structure 50. In the embodiment shown in FIGS. 1-4, the secondary absorbent structure 52 lies beneath the primary absorbent structure 50. The secondary absorbent structure 50 can comprise any type of absorbent material that is suitable for these purposes. In the preferred embodiment shown in FIGS. 1-4, the secondary absorbent structure 52 comprises a high capacity thermally bonded airlaid nonwoven material fabricated from a blend of cellulose and bicomponent fibers (referred to as "TBAL" material for brevity). The TBAL material preferably comprises a homogeneous blend of about 55% Flint River fluff (cellulose), 34% Nalco 1180 absorbent gelling material particles obtained from Nalco of Naperville, IL, and 11% DANKLON ES-C 3.3 dtex x 6 mm bicomponent fibers obtained from Dan Web of Aarhus, Denmark. The TBAL material is formed into a web having a basis weight of about 210 grams/m², and a caliper of about 1.6 mm measured under a load of about 1.2 g/cm².

The secondary absorbent structure 52 is preferably covered by an optional intermediate acquisition component 66. The optional intermediate acquisition component 66 serves to drain liquids through the topsheet 44 and provide void volume so that the underlying TBAL layer (which has high storage capacity, but relatively slow rate of absorbency) will have time to absorb bodily exudates deposited thereon. The optional acquisition component 66 is, thus, more important for use with the portion of the secondary absorbent structure 52 that lies outside the boundaries of the primary absorbent structure 50. The acquisition component 66 can comprise any material which is suitable for the aforementioned purposes. In the preferred embodiment shown in FIGS. 1-4, the acquisition component 66 comprises a layer of an 18 g/yd² (21.5 g/m²) spun bonded polypropylene nonwoven material known as CELESTRA available from Fiberweb, North America of Simpsonville, SC, which is embossed with the pattern described in U.S. Patent 4,781,710 issued to Megison, et al. on November 1, 1988. The acquisition component is preferably of the same shape and size as the TBAL layer. The CELESTRA material is preferably bonded to the overlying component (which will either be the primary absorbent structure 50 or, for the portions of the acquisition component 66 that lie outboard of the primary absorbent structure, the underside of the hybrid topsheet 44), but is unbonded to the TBAL layer.

In an alternative embodiment, the positions of the absorbent materials can be reversed so that the TBAL material overlies the airfelt component. In such a case, the TBAL layer could be considered to comprise the primary absorbent structure, and the airfelt component could be considered to comprise a secondary absorbent structure that provides a portion of the sanitary napkin 20 with additional bulk. In other alternative embodiments, such a bulking material can comprise any other suitable absorbent materials, or even nonabsorbent materials. One suitable absorbent material comprises a carded or airlaid thermal air through bonded nonwoven material comprised of bicomponent fibers having an eccentric cross-section that has a wetting agent included in the sheath resin and a permanent wetting agent applied to the surface which is described in greater detail in U.S. Patent 5,231,122 issued to Palumbo, et al. Suitable nonabsorbent materials can comprise closed cell foams, polyurethane foams, and high loft nonwoven webs comprised of synthetic fibers.

The backsheet 46 prevents the exudates absorbed by and contained in the components of the absorbent core 48 from wetting articles which contact the sanitary napkin 20 such as the wearer's pants, pajamas and undergarments. The backsheet 46 should be flexible and impervious to liquids (e.g. menses and/or urine). The backsheet 46 may comprise a woven or nonwoven material, polymeric films such as thermoplastic films of polyethylene or polypropylene, or composite materials. Preferably, the backsheet 46 comprises a polyethylene film having a thickness of from about 0.012 mm (0.5 mil) to about 0.051 mm (2.0 mils). An exemplary polyethylene films is a product known as microflex 1401 manufactured by The Clopay Corporation of Cincinnati, Ohio.

The topsheet 44, the backsheet 46, and the absorbent core 48 may be assembled in a variety of configurations known in the art (including so called "sandwich" products and "tube" products). Several preferred sanitary napkin configurations and features that the sanitary napkin can be provided with are described generally in U.S. Patent 4,321,924, "Bordered Disposable Absorbent Article" issued to Ahr on March 30, 1982; U.S. Patent 4,425,130, "Compound Sanitary Napkin" issued to DesMarais on January 10, 1984; U.S. Patents 4,950,264 and 5,009,653, both entitled "Thin, Flexible Sanitary Napkin" issued to Osborn on August 21, 1990 and April 23, 1991, respectively; and the aforementioned patent applications issued to Sneller, et al. The main body portion 22 of the sanitary napkin may also be comprised of one or more extensible components and may either have an overall extensibility, regions with extensibility, such as those sanitary napkins, and the like described in U.S. Patent Application Serial Nos. 07/915,133 and 07/915,284 both filed July 23, 1992, in the name of Osbom, et al. (PCT Publication Nos. WO 93/01785 and 93/01786, both published February 4, 1993).

The sanitary napkin 20, as shown in.FIGS. 2 and 4, is preferably assembled in a sandwich construction in which the topsheet 44 and the backsheet 46 have dimensions that are generally larger than those of the absorbent core 48. FIG. 4 shows the assembly of the components of the sanitary napkin 20. As shown in FIG 4, the hybrid topsheet 44 is joined to the primary absorbent structure 50 in the region of the sanitary napkin that includes the primary absorbent structure 50. The hybrid topsheet 44 is joined to the optional intermediate acquisition component 66 in the region of the sanitary napkin that lies outboard of the primary absorbent structure 50. The hybrid topsheet 44 is joined to the backsheet 46 in the region of the sanitary napkin that lies outboard of the absorbent core 48. Preferably, the hybrid topsheet 44 is joined to these components by a core bonding adhesive that is applied in a spiral pattern. The secondary absorbent structure 52 is preferably joined to the backsheet 46. Preferably, the secondary absorbent structure 52 and the backsheet 46 are joined using a core integrity adhesive 70 applied in a plurality of strips of adhesive, each of which comprises spirals of adhesive. Exemplary means for joining these components of the sanitary napkin 20 comprises several lines of adhesive filaments swirled into a spiral pattern such as illustrated by the apparatus and method shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. The core integrity adhesive 70 can be applied over the entire garment facing side of the secondary absorbent structure 52, over the whole product width (including the extensions of the backsheet that will lie beyond the edges of the secondary absorbent structure) or any portion thereof. Preferably, the core integrity adhesive 70 is applied to the entire interface between the garment facing side of the secondary absorbent structure 52 and the backsheet 46.

In addition to joining the faces of the components of the sanitary napkin 20 as described above, FIG. 1 shows that the topsheet 44 and backsheet 46 are joined together to form a seam 72 along at least portions of the periphery 30 of the main body portion. The seam 72 can be formed by any means commonly used in the art for this purpose, such as gluing, crimping, or fusing. In the preferred embodiment shown in FIG. 1, the seam 72 is formed by a plurality of rectangular spaced apart fusion bonds 72A (only a few of which are shown for simplicity). In addition, longitudinally oriented fusion bonds 72B are applied to the central region of the side wrapping elements 24 along the distal edge 76 of the same. It is to be understood that the embodiment illustrated in the drawings is only one possible embodiment, albeit a preferred one. Other possible embodiments include one in which an absorbent core 48 is essentially completely wrapped with a topsheet before it is placed on a backsheet. The main body portion 22 can also comprise an absorbent core which possesses sufficient integrity to stand alone and is liquid pervious on one surface while the other surface has been treated to render it liquid impervious.

The sanitary napkin 20 of the present invention comprises a pair of side extensions (or "side wrapping elements") 24 for folding around the side edges of the wearer's panties (or other undergarment). As shown in FIG. 1, the main body portion 22 is narrower in width measured across its central region 36 than at its end regions 32 and 34. The side wrapping elements 24 extend from at least the central region 36 of the main body portion 22. The side wrapping elements 24 are preferably configured so that the majority of the surface area of the side wrapping elements 24 is located laterally inward of the laterally outwardmost portion 40 of the main body portion 22. The sanitary napkin 20 can thus be thought of as having "internal flaps" that can fold around a wearer's undergarments.

The side wrapping elements 24 each have a proximal edge 74 and a distal edge 76. In the embodiment shown in the drawings, the proximal edges 74 of the side wrapping elements 24 are preferably concave (relative to the distal edges 76). The distal edges 76 of the side wrapping elements 24 are preferably approximately parallel to the longitudinal centerline L. The sanitary napkin 20 may be thought of as having "internal flaps" because the side wrapping elements 24 are longitudinally inboard of the outermost edges of the lobes 38 of the main body portion 22 and the distal edges 76 of the side wrapping elements 24 preferably do not extend appreciably laterally outward beyond the outermost edges of the lobes 38 of the main body portion 22 of the sanitary napkin 20 and any peripheral flange, such as seam 72 around the same.

The side wrapping elements 24 are joined to the main body portion 22 at their proximal edges 74. The term "joined", as used herein, encompasses configurations in which an element is directly secured to another element by affixing the element directly to the other element; configurations in which the element is indirectly secured to the other element by affixing the element to intermediate member(s) which in turn is affixed to the other element; and configurations in which one element is integral with another element, i.e., one element is essentially part of the other element.

The side wrapping elements 24 of the embodiment shown in FIGS. 1-3 are preferably integral with the main body portion 22 of the sanitary napkin. In such a case, the topsheet 44 may form a portion of the side wrapping elements 24 and the backsheet 46 may also form a portion thereof. For example, the topsheet 44 may form the body-facing surface of both the side wrapping elements 24 and the main body portion 22, and the backsheet 40 may form the garment-facing surface of the same. It is also possible for the absorbent material of the sanitary napkin 20 to extend into the side wrapping elements 24, as described in greater detail for the side flaps of the sanitary napkin in U.S. Patent 4,917,697. In alternative embodiments, the side wrapping elements 24 may be comprised of separate pieces of material or elements which are attached to the main body portion 22. The side wrapping elements 24 may be joined in any of the manners that the side flaps are joined to the absorbent article described in U.S. Patent 5,389,094 issued to Lavash, et al. on February 14, 1995. When the side wrapping elements 24 comprise separate elements, they can be joined to the main body portion 22 by any techniques known to those skilled in the art. Such techniques include, but are not limited to adhesives, heat and/or pressure, ultrasonics, etc.

The side wrapping elements 24, whether they are integral with the main body portion or separate elements attached thereto, are each associated with main body portion 22 along a juncture. The juncture is typically a longitudinally-oriented (or "longitudinal") juncture, such as line of juncture 78. As used herein, the terms "juncture" (or "line of juncture") refer to regions where the side wrapping elements 24 extend from or are joined to the main body portion 22. The junctures 78 can be any of various curved or straight lines, but they are not limited to lines. Thus, the junctures can comprise regions, flanges, strips, intermittent lines, and the like. In the sanitary napkin 20 illustrated in FIG. 1, line of juncture 78 is a generally longitudinally oriented region that is concave relative to the distal edges 76 of the side wrapping elements. When the side wrapping elements 24 are integral with the main body portion 22, the lines of juncture 78 may represent lines of demarcation between the main body portion 22 and the side wrapping elements 24, although it is not necessary that there be a precise line of demarcation.

The side wrapping elements 24 are preferably more flexible (that is, less stiff) than those parts of the main body portion that form the longitudinal side edges 26 of the main body portion. The difference in stiffness along the longitudinal side edges 26 of the main body portion 22 provides the sanitary napkin 20 with a curved hinge line about which the side wrapping elements 24 may fold.

As shown in Figure 1, each side wrapping element 24 is divided into a front half 80, and a back half 82 by a side wrapping element transverse centerline T₁. The side wrapping element transverse centerline T₁ may coincide with the principal transverse centerline T of the sanitary napkin, but this is not absolutely required. In other embodiments where the main body portion 22 is not symmetrical along its length, the side wrapping elements 24 may be located more toward one end of the main body portion, and the side wrapping element transverse centerline T₁ may, thus, be offset either to the front or to the rear of the principal transverse centerline T.

The side wrapping elements 24 are provided with weakened regions 84 that are more flexible than the adjacent regions 86 of the side wrapping elements. The weakened regions 84 are located so that on each side wrapping element 24, at least one weakened region, or portion thereof, lies on each side of the side wrapping element transverse centerline T₁. The weakened regions 84 are preferably at least partially disposed longitudinally away from the flap transverse centerline T₁ in both directions. (Thus, the weakened regions 84 may be described as being longitudinally "remote" from the side wrapping element transverse centerline T₁. In the most preferred case (as will be subsequently described in greater detail), the weakened regions 84 are located along a portion of the fold line where the side wrapping elements 24 are folded around the wearer's panty crotch. The fold line will typically be located along or adjacent the longitudinal juncture 78 of each side wrapping element 24. Since the terms "portions", "zones", and "regions", as used herein, refer to general areas, the weakened regions 84 are, thus, not limited to points which lie precisely on the line of juncture 78. Typically, they will include both those points which lie on the lines of juncture 78 as well as the surrounding areas of the sanitary napkin 20 which include the aforementioned fold lines). The longitudinal junctures, thus, may merely serve as approximations for the location of the weakened regions 84.

The weakened regions 84 are preferably also extensible. The weakened regions 84 may, thus, be thought of as comprising zones of differential extensibility (or "zones of extensibility"). The term "zones of differential extensibility", as used herein, refers to a portion of the side wrapping element 24 which is capable of extending a differing amount (preferably a greater amount), than adjacent regions 86 of the side wrapping element 24. The extensibility of the weakened regions 84 relieves the stresses which develop in the side wrapping elements 24 when they are folded around the sides of the wearer's panty crotch.

The weakened regions 84 are preferably primarily extensible generally outward in the transverse direction. As used herein, the phrase "generally in the transverse direction" means that the extensibility has a transverse component. All of the extension, however, need not be exactly parallel to the principal transverse centerline, T, of the sanitary napkin. For example, in the embodiment shown in FIG. 1, the weakened regions 84 are extensible in a direction between the longitudinal and transverse directions. The extensibility of the weakened regions 84, however, is preferably oriented more in the transverse direction than in the longitudinal direction so that it is still generally in the transverse direction. Although, it is also possible that in other embodiments, the extensibility of the weakened regions 84 can be oriented more in the longitudinal direction than the transverse direction, or even entirely in the longitudinal direction.

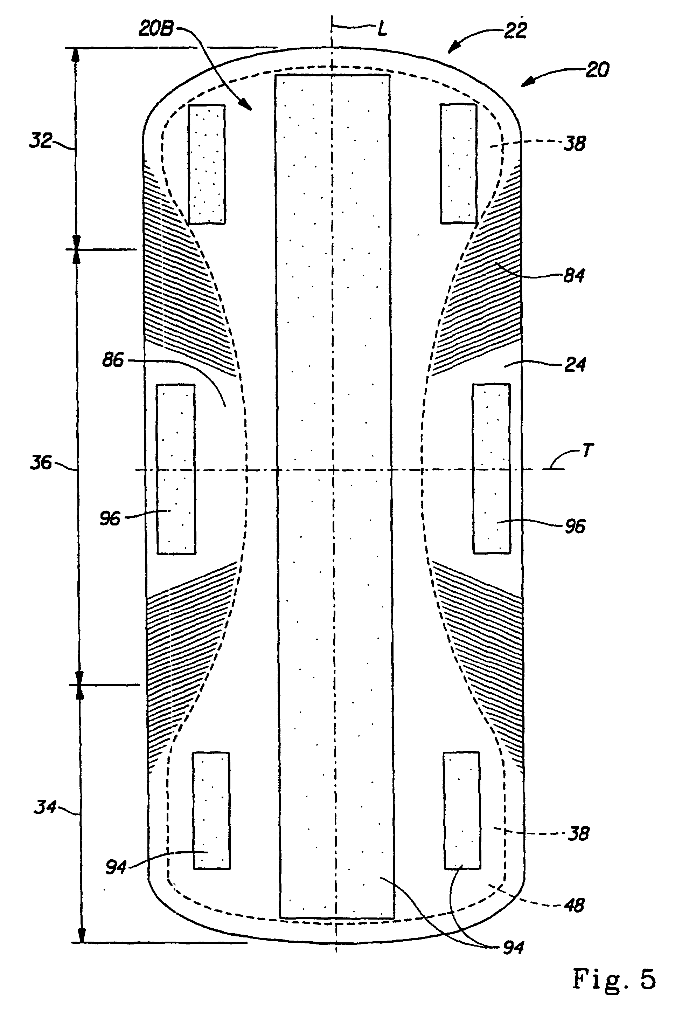

The weakened regions 84 can comprise any structure that is more flexible and extensible than the adjacent regions 86 of the side wrapping elements 24. Suitable structures for the weakened regions 84 include, but are not limited to zones of material that are mechanically strained, corrugated, "ring rolled" (the term "ring rolled" refers to a straining/activation achieved by feeding a material through intermeshing corrugated rolls), folded, formed into a Structural Elastic-Like Film (or "SELFed" structure) as described in allowed U.S. Patent Application Serial No. 08/203,087 entitled "Web Materials Exhibiting Elastic-Like Behavior", filed in the name of Chappel, et al. on February 28, 1994, to be issued as U.S. Patent 5,518,801 on May 21, 1996 (PCT Publication No. WO 95/03765, published February 9, 1995) and in U.S. Patent Application Serial No. 08/124,180 filed by Mansfield, et al. (PCT Publication No. WO 94/10200), or pleated, or joined along a curved juncture. These weakened regions 84 (although shown in FIGS. 1-3 as only being part of the side wrapping elements 24), can comprise portions of the main body portion 22, portions of the side wrapping elements 24, or both. Examples of sanitary napkins having flaps and zones of differential extensibility are further described in U.S. Patent 5,354,400 issued to Lavash, et al. on October 11, 1994, and U.S. Patent 5,389,094 issued to Lavash, et al. on February 14, 1995. Other, but less preferred, examples of structures that can provide the side wrapping elements 24 with a degree of flexibility and extensibility are the notches shown in FIG. 5 of U.S. Patent B1 4,589,876 issued to Van Tilburg and the stress relief means described in U.S. Patent 4,917,697 issued to Osborn, et al.

The sanitary napkin 20 shown in FIGS. 1-3 has side wrapping elements 24 that have been provided with weakened regions 84 by ring rolling the desired regions of the side wrapping elements 24. The weakened regions 84 are ring rolled in accordance with methods described in U.S. Patent 4,107,364 issued to Sisson on August 15, 1978, U.S. Patent 4,834,741 issued to Sabee on May 30, 1989, U.S. Patent 5,143,679 issued to Gerald M. Weber, et al. on September 1, 1992, U.S. Patent 5,156,793 issued to Kenneth B. Buell, et al. on October 20, 1992, and U.S. Patent 5,167,897 issued to Gerald M. Weber, et al. on December 1, 1992. The ring rolling (also known as "pre-corrugating") forms corrugations in the weakened regions 84. The corrugations comprise ridges and valleys that are defined by fold lines 88. The fold lines 88 may form any angle desired relative to the principal longitudinal centerline L. In the preferred embodiment shown in FIGS. 1-3, the fold lines 88 form an angle of between about 40° - 45° with the principal longitudinal centerline L. This will provide the desired direction of extensibility.

The side wrapping elements 24 are sufficiently flexible and are sized and configured so that they are capable of folding around the side edges of a crotch region of a wearer's undergarment. In order to be capable of folding around the sides of an undergarment, the side wrapping elements 24 must be of a certain minimum size (that is, length and width). Otherwise, the adjacent stiffer portions of the sanitary napkin 20, such as the lobes 38, of the main body portion 22, to which the side wrapping elements 24 are joined, will restrict and prevent the side wrapping elements 24 from folding. The side wrapping elements 24 preferably range in size from about 2 cm in width (transverse direction from their proximal edge to their distal edge) and about 16.5 cm in length (longitudinal direction) up to about 4.5 or 5 cm in width and about 23.5 cm in length. The side wrapping elements 24 in the embodiment shown in FIGS. 1-3 are preferably about 2 inches (about 5 cm) in width from their proximal edge to their distal edge, and about 8 inches (about 20 cm) in length, and measure greater than or equal to about 160 mm, more preferably greater than or equal to about 170 mm, more preferably greater than or equal to about 180 mm, more preferably still greater than or equal to about 190 mm, and most preferably greater than or equal to about 200 mm along their curvilinear proximal edge.

The enhanced flexibility and extensibility of the weakened regions 84, along with the difference in flexibility between the side wrapping elements 24 and longitudinal side edges 26 of the main body portion 22, allows the side wrapping elements 24 to fold smoothly around the edges of the wearer's panties. If the longitudinal side edges 26 of the main body portion 22 are approximately the same size and shape as the side edges of the wearer's panties, the side wrapping elements 24, in a most preferred embodiment, can fold on a curvilinear line virtually exactly along the side edges of the wearer's panties. Preferably, the side wrapping elements 24 fold at least along a generally curvilinear line that lies at least generally along the side edges of the wearer's panties. The side wrapping elements 24 can, if desired, bend through an angle of 180 degrees, and be attached to the underside of the wearer's panties. The fact that the side wrapping elements 24 can fold along a curvilinear line, allows the side wrapping elements to form a flat fold along the length of the edges of the wearer's panties. This reduces the tendency for the side wrapping elements to bunch longitudinally inward which would reduce the area of the wearer's panties the side wrapping elements 24 are able to cover. It also reduces the tendency for the ends of the main body portion along the longitudinal side edges thereof to become detached from the wearer's panties and lift up and fold over onto the topsheet. It further substantially reduces, if not eliminates, any tendency for the side wrapping elements 24 to become unattached to the underside of the wearer's panties (or for the fastener on the side wrapping elements to "pop" off from their attachment with the underside of the panties).

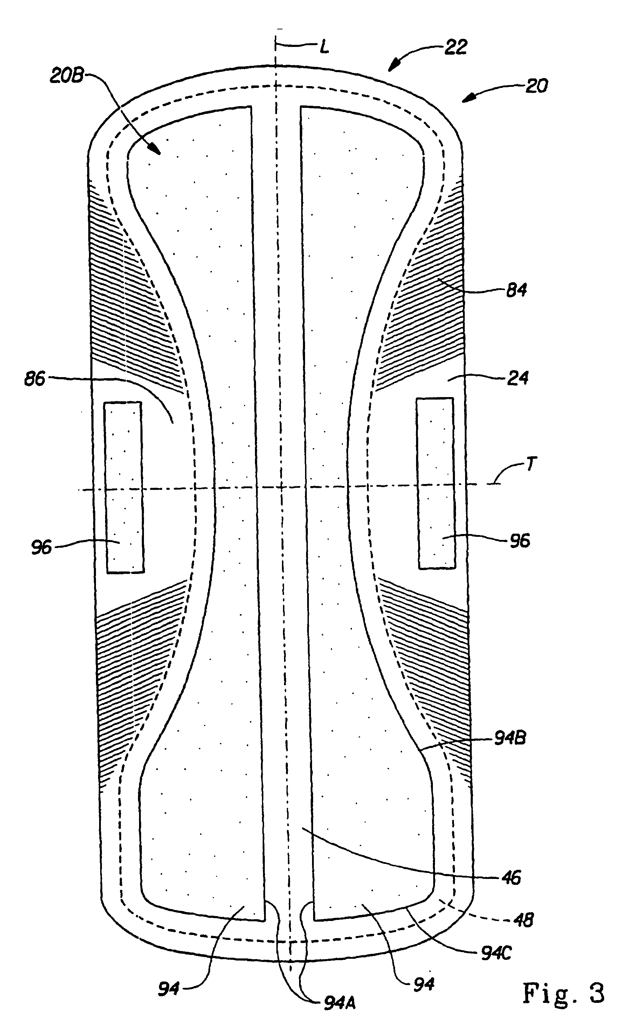

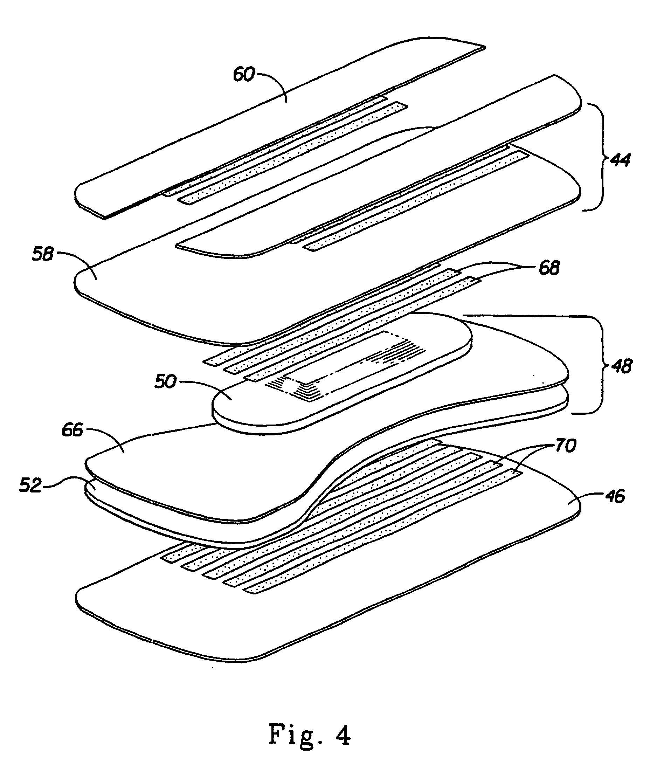

The fact that the side wrapping elements 24 of the present invention can bend through an angle of 180 degrees also distinguishes the side wrapping elements from the flaps of the sanitary napkin described in U.S. Patent 4,790,838 issued to Pigneul, et al. The Pigneul, et al. reference is not directed to a sanitary napkin having flaps that are of a side wrapping type. The Pigneul, et al. reference is directed to a sanitary napkin having a sealed area for ensuring "increased lateral tightness with a view to preventing any lateral leakage of fluids". There is nothing in the Pigneul, et al. reference to indicate that the sealed portions of the sanitary napkin described therein are capable of folding around a wearer's undergarments. For example, Fig. 3 of Pigneul shows that the flaps of the Pigneul sanitary napkin have a span that is roughly only as large as the thickness of the absorbent element. This, combined with the small area of the flaps shown in plan view in Fig. 1 of Pigneul makes it apparent that there is not enough material in these flaps to wrap the sides of a pair of panties. Thus, it is clear that the flaps of the sanitary napkin described in the Pigneul reference are merely intended to extend laterally outward, and the pleats therein are intended to form barriers or dams to prevent the flow of liquids in the lateral direction. There is also nothing in the Pigneul reference to indicate that the pleated portions of the flaps that are extensible. In fact, the way in which the flaps are described as being assembled in the Pigneul, et al. reference (the pleats are sealed together as described in Col. 3, lines 51-56) of Pigneul shows that they would not tend to be extensible.

The side wrapping elements 24 of the present invention differ in a number of respects from conventional wings or side flaps that have been previously used on sanitary napkins. First, the side wrapping elements 24 need not extend outward appreciably beyond the laterally outwardmost edge of the main body portion 22. This is in contrast with conventional side flaps which need to extend substantially laterally outward beyond the laterally outwardmost edge of their main body portion in order to function properly. Second, the side wrapping elements 24 can have generally rectilinear distal edges 76 and weakened regions 84 that allow the side wrapping elements 24 to fold smoothly along at least one curvilinear axis. The side wrapping elements 24 are, thus, able to cover a large area along the elasticized side edges of the wearer's panties. The side wrapping elements 24 are preferably able to cover a length of the panty elastics that runs substantially the entire length of the concave portion of the longitudinal side edges 26 of the main body portion 22. Preferably, the side wrapping elements are able cover a length measured along the elasticized sides of the wearer's panties of greater than or equal to about 160 mm, more preferably greater than or equal to about 170 mm, more preferably greater than or equal to about 180 mm, more preferably still greater than or equal to about 190 mm, and most preferably greater than or equal to about 200 mm.

The side wrapping elements 24 can be formed in any suitable manner. For example, in one version of the embodiment shown in FIGS. 1-3, areas for the side wrapping elements 24 can be formed by cutting the secondary absorbent structure 52 at certain places and then folding portions of the secondary absorbent structure inward over the central region of the same. As shown in FIG. 4A, a pair of curvilinear slits 90 are formed in the secondary absorbent structure 52. The slits 90 extend from points on the longitudinal side edges of the secondary absorbent structure 52 toward, but not all the way to, the transverse centerline thereof. The outer portions 92 of the secondary absorbent structure that are outboard of the slits are then folded inward as shown in Fig. 4B. This leaves a cut out area having a curvilinear edge 93. The curvilinear edge 93 is preferably at least about 160 mm long and the outer portion 92 formed by the cutting is preferably about 2 inches in width and about 8 inches long. The portions 92 of the secondary absorbent structure that are folded can be folded either over or under the central region of the secondary absorbent structure 52. These outer portions 92 can also be folded either over or under the primary absorbent structure 50.

The sanitary napkin 20 is formed by adding a rectangular topsheet and backsheet, and joining the components of the sanitary napkin as described above. In the cutout region, the topsheet 44 and backsheet 46 are preferably at least partially secured together by adhesives. Since the topsheet 44 and backsheet 46 comprise generally rectangular sheets, this provides the advantage that the sanitary napkin will use the entire topsheet and backsheet materials without requiring any portions thereof to be removed and thrown away as scrap as is generally done when making sanitary napkins with outwardly extending side flaps that are integral with the topsheet and backsheet. (Ordinarily, when making sanitary napkins with conventional outwardly extending flaps, the material that is cut around the flaps will be scrapped.) The method of making the sanitary napkin described above also provides additional absorbency without the need to throw away portions of the secondary absorbent structure as scrap. Alternatively, the folded portions of the secondary aborbent structure 52 could provide the sanitary napkin with all the absorbency desired without adding a separate primary absorbent structure.

The sanitary napkin 20 preferably also has fasteners that are adapted to secure the sanitary napkin 20 to the crotch region of an undergarment. FIGS. 2 and 3 show one preferred type of fastener, in the form of an adhesive attachment means, such as central pad adhesive 94 and side wrapping element adhesive 96. The fasteners used with the sanitary napkin of the present invention are, however, not limited to adhesive attachment means. Any type of fastener used in the art can be used for such purpose. For example, the sanitary napkin 20 could be secured to the wearer's undergarment by frictional fasteners, mechanical fasteners, or a combination of any of the foregoing types of fasteners. For simplicity, however, the fasteners will be described in terms of adhesive attachment means and are preferably pressure sensitive adhesive fasteners. Suitable pressure sensitive adhesive fasteners are described in greater detail in U.S. Patent 4,917,697.

The adhesive fasteners 94 and 96 can be arranged in any suitable configuration. FIG. 3 shows one possible panty fastener pattern. The panty fastener pattern shown in FIG. 3 comprises a pair of longitudinally-oriented central pad fasteners 94 that lie on opposite sides of the principal longitudinal centerline L. The longitudinally-oriented central pad fasteners 94 shown in FIG. 3 preferably extend substantially the entire length of the absorbent core 48. The longitudinally-oriented central pad fasteners 94 preferably each have an inside edge 94A which is generally linear. The inside edges 94A of the longitudinally-oriented fasteners 94 are preferably spaced away from each other and from the principal longitudinal centerline L of the sanitary napkin 20. This allows a longitudinally-oriented central region of the sanitary napkin 20 (that does not have a fastener thereon) to move apart from the wearer's panties and move into close contact with the wearer's body. The longitudinally-oriented central pad fasteners 94 preferably have outside edges 94B and ends 94C that are shaped similarly to the outer edges of the absorbent core 48. This provides a central pad fastener 94 that is generally hourglass shaped with a longitudinally oriented gap in the center. In addition to the longitudinally oriented central pad fasteners 94, the sanitary napkin 20 preferably has a rectangular side wrapping element fastener 96 on each side wrapping element 24 which lies along the transverse centerline T of the sanitary napkin 20.

FIG. 5 shows a sanitary napkin 20 having another possible panty fastener pattern. The panty fastener shown in FIG. 5 comprises a central pad fastener 94 that comprises a longitudinally-oriented rectangular zone that is centered along the longitudinal centerline L of the sanitary napkin 20 and extends approximately the entire length of the absorbent core 48. The sanitary napkin 20 also preferably comprises a pair of spaced apart rectangular patches of fastener material in each end region, 32 and 34, of the sanitary napkin. The pairs of spaced apart rectangular patches in the end regions preferably comprise one patch adjacent each edge of the lobes 38 formed by the absorbent core 48. In addition, as in the case of the previous embodiment, the sanitary napkin 20 preferably also comprises a rectangular side wrapping element fastener 96 on each side wrapping element 24 which lies along the transverse centerline T of the sanitary napkin 20.

The adhesive attachment means, such as the central pad adhesive 94 and the side wrapping element adhesive fasteners 96, may each be covered by separate removable release liners to keep the adhesives from sticking to extraneous surfaces prior to use. A suitable release liner that can be used for the side wrapping element fasteners 96 is described in U.S. Patent Application Serial No. 08/247,912 filed May 23, 1994, entitled "Absorbent Article Having Flaps With Unitary Release Strip" in the name of Osborn, which was originally filed June 5, 1990 (PCT Publication No. WO 91/18574, published December 12, 1991). Preferably, however, both of the adhesive attachment means are covered by an arrangement wherein at least one of the release liners comprises a releasable wrapper 100 that also serves as an individual package for the sanitary napkin. Suitable release liners that serve as an individual package for a sanitary napkin are described generally in U.S. Patent 4,556,146 issued to Swanson, et al. (which discloses a tri-folded sanitary napkin and wrapper). The sanitary napkin 20 described herein, however, is considerably larger than most sanitary napkins currently in use. Because of its unusual shape and panty fastener configuration, particularly the panty fastener configuration shown in FIG. 5, the sanitary napkin 20 requires a novel version of such releasable wrappers in order to form a package which is compact enough to be conveniently carried by a consumer.

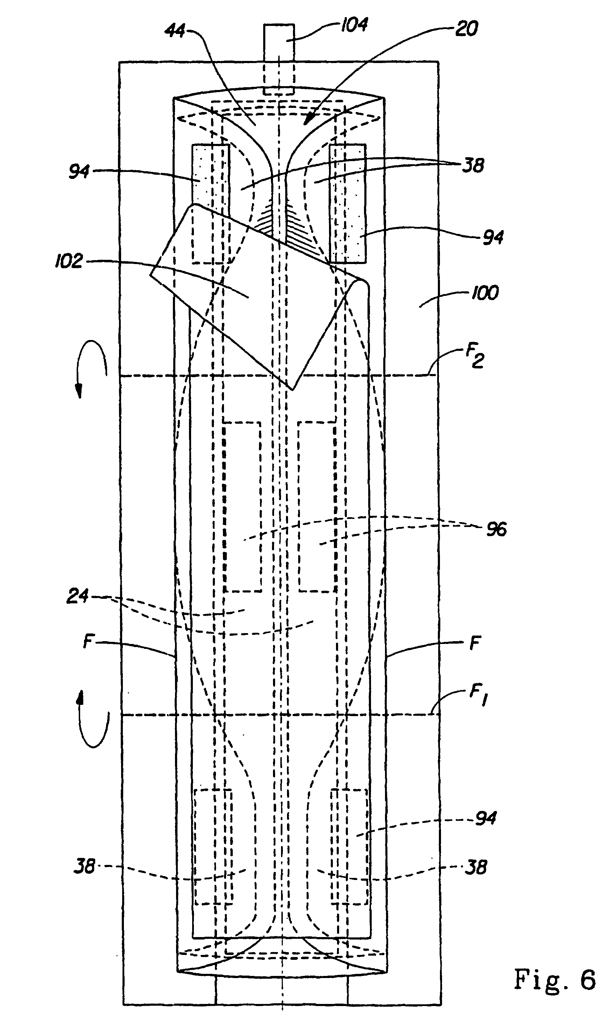

FIG. 6 shows the sanitary napkin 20 after it has been folded and placed on a releasable wrapper 100 that will serve as an individual package for the sanitary napkin 20. The lobes 38 and side wrapping elements 24 of the sanitary napkin 20 are preferably folded inward along longitudinal fold lines, F, over the topsheet 44. This exposes the pairs of adhesive patches 94 in the end regions of the sanitary napkin and the adhesive side wrapping element fasteners 96. The sanitary napkin 20 is placed garment surface 20B down on the releasable wrapper 100 so that the central pad fastener 94 is releasably attached to the releasable wrapper 100. The exposed pairs of adhesive patches 94 in each end region of the sanitary napkin and the side wrapping element fasteners 96 are then covered with a single sheet of strip of release paper 102. The sanitary napkin 20 and the releasable wrapper 100 are then preferably folded about a pair of spaced apart transverse axes F₁ and F₂ to form an individual package for the sanitary napkin 20. The releasable wrapper 100 is preferably provided with a releasable adhesive wrapper closure fastener, such as adhesive tape tab 104, for retaining the folded sanitary napkin and wrapper in their folded configuration.

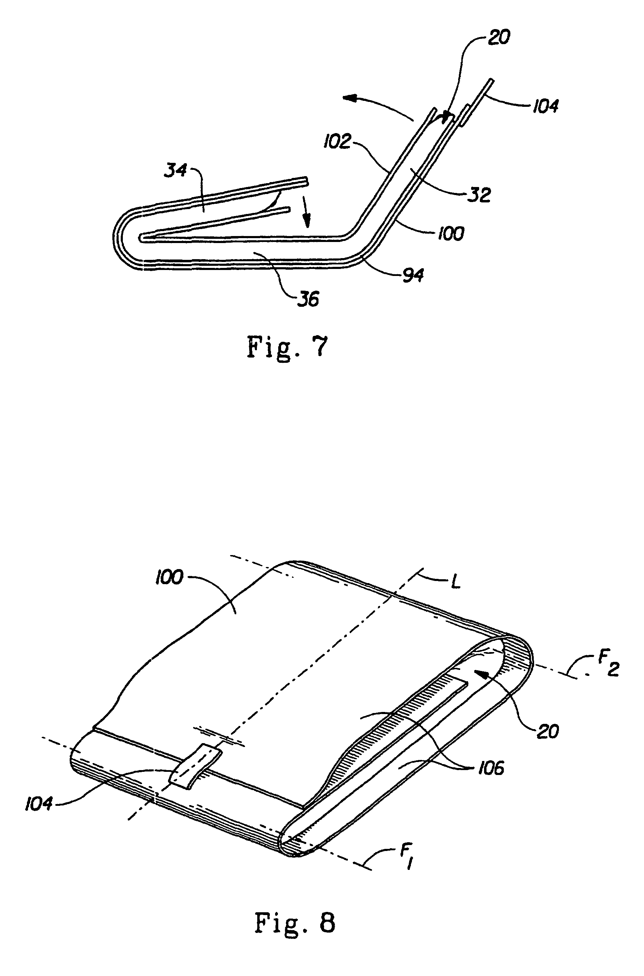

FIGS. 7 and 8 show the folding of the sanitary napkin 20 and releasable wrapper 100 to form an individual package for the sanitary napkin. FIG. 7 shows that the second end region 34 of the sanitary napkin 20 is folded over the central region 36 of the sanitary napkin 20. The first end region 32 of the sanitary napkin 20 is then folded on top of the second end region 34. FIG. 8 shows that the adhesive tape tab 104 is then used to releasably secure the sanitary napkin 20 in its folded configuration. In a particularly preferred embodiment, the longitudinal edges 106 of the releasable wrapper 100 extend beyond the side edges of the folded sanitary napkin 20. The longitudinal edges 106 of the releasable wrapper 100 are preferably frangibly sealed together to close off the sides of the package. Suitable methods for frangibly sealing the longitudinal edges 106 of such a package are described in U.S. Patent 4,556,146 issued to Swanson, et al., U.S. Patent 5,181,610 issued to Quick, and U.S. Patent 5,462,166 issued to Minton, et al.

The folded configuration shown in FIGS. 6-8 provides the advantage that it produces a relatively small and convenient package for a relatively large pad. The folded package preferably has overall dimensions of about 5.5 inches (about 14 cm) measured in the longitudinal direction, about 5 inches (about 13 cm) in width, and less than or equal to about 1 inch (2.5 cm) in thickness.

The sanitary napkin 20 is removed for use by peeling open the tape tab 104 then unfolding the end regions of the sanitary napkin 20 in the reverse order that they were originally folded to package the sanitary napkin 20. This breaks the frangible seals along the longitudinal side edges 106 of the releasable wrapper 100 and places the sanitary napkin 20 and releasable wrapper 100 in the flat, laid out configuration shown in FIG. 6. The consumer can then peel away the releasable wrapper 100 from the central body fastener 94. The consumer can thereafter place the sanitary napkin 20 in the crotch region of her panties and can adjust the sanitary napkin until it is in the desired position. This can all be done while the strip of release paper 102 still covers the fasteners on the lobes 38 of the sanitary napkin and on the side wrapping elements 24. This provides the advantage that the fasteners 94 and 96 on these portions of the sanitary napkin 20 will not be able to fold over on themselves and stick to themselves or to the wrong portion of the wearer's panties. The consumer can then peel back the release strip 102 and unfold the lobes 38 and the side wrapping elements 24. The consumer can then secure the lobes 38 to her panties and fold the side wrapping elements 24 around the edge of her panties and attach the side wrapping element fasteners 96 to the underside of her panties.

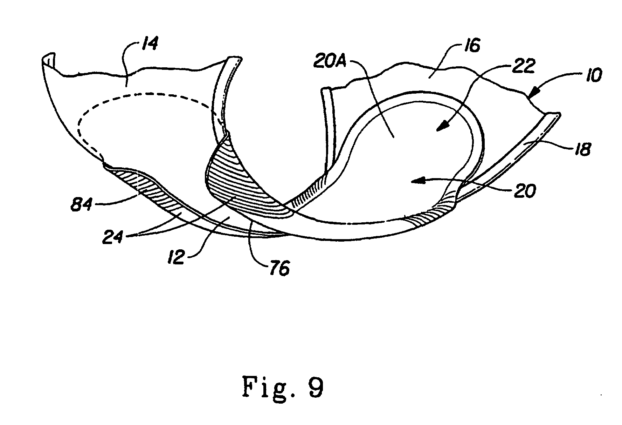

Figure 9 is a depiction of the sanitary napkin 20 of the present invention in place in an undergarment of the type commonly worn by many women and well known as a panty 10. The configuration of the sanitary napkin 20 in the panty shown in FIG. 9 is presented primarily for purposes of discussion, rather than to limit the possible configurations the sanitary napkin may take in use. It should be understood that the sanitary napkin 20 described herein may also take other configurations in use. For example, the side wrapping elements of the sanitary napkin 20 can, if desired, take in-use configurations similar to those of the flaps described in U.S. Patent 4,687,478 and 5,267,992 issued to Van Tilburg or U.S. Patent 5,354,400 issued to Lavash, et al. Of course, the in-use configuration will differ somewhat since the span of the side wrapping elements 24 will typically be less than such flaps.

The panty 10 comprises a crotch portion 12, a front section 14, and a back section 16. The crotch portion 12 joins the front and back sections and comprises two elasticized side edges 18. As shown in Figure 9, the center of main body portion 22 is placed in the crotch portion 12 of the panty 10 with the backsheet 46 in contact with the inner surface of crotch portion 12 of the panty and one end of main body portion 22 extending towards the front section 14 of the panty and the other end towards the back section 16. Central pad adhesive 94 maintains main body portion 22 in position. The distal portions 76 of side wrapping elements 24 are folded around the elasticized side edges 18 of the panty. The flap adhesive portions 96 secure the side wrapping elements 24 to the underside of the panty.

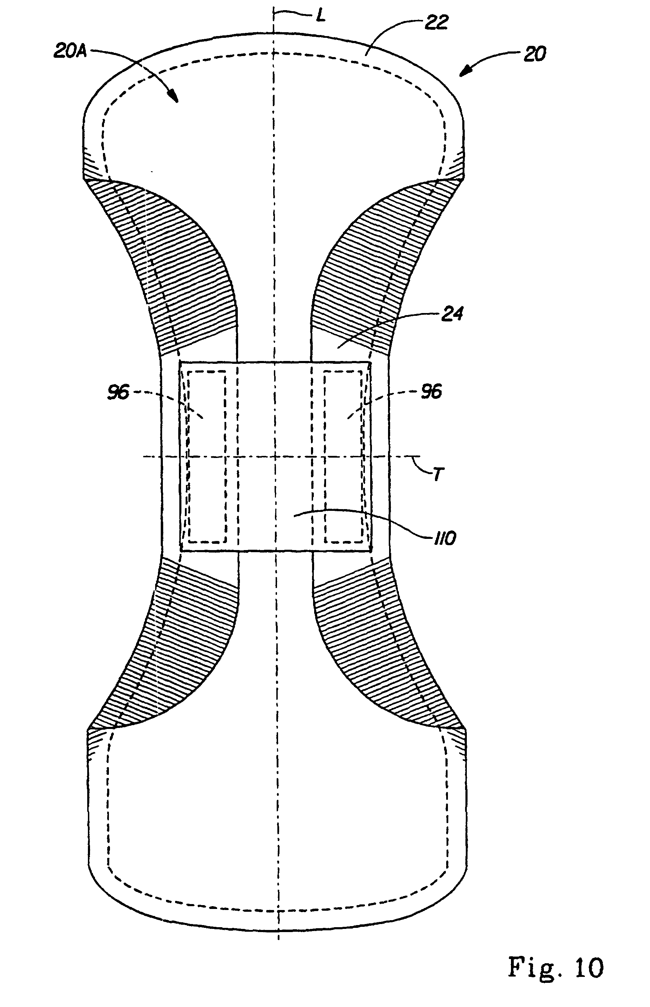

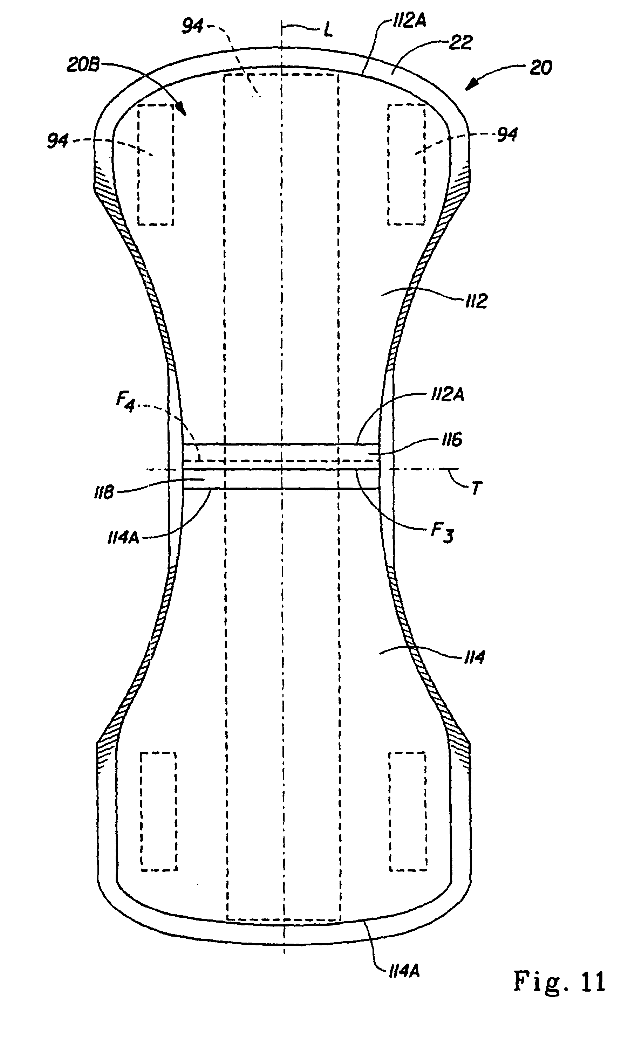

FIGS. 10 and 11 show an alternative way of covering the adhesive fasteners on the garment facing side of the sanitary napkin. As FIG. 10 shows, in this embodiment, the side wrapping elements 24 are folded over the body-facing side 20A of the main body portion 22 and the adhesive fasteners 96 thereon are covered with a unitary release strip 110. As shown in FIG. 11, the adhesive fasteners 94 on the garment-facing side 20B of the main body portion 22 are covered with multiple release papers such as release papers 112 and 114. Preferably, these multiple release papers 112 and 114 are oriented in an end-to-end relationship in the longitudinal direction. Each of the release papers 112 and 114 generally resembles the shape of half of an hourglass. The release papers 112 and 114 preferably have a portion adjacent at least one edge, which is preferably an end edge, such as 112A) that is. non-adhesive, that overlaps with a portion adjacent the end edge (such as 114A) of the adjacent release paper. In the preferred embodiment shown, the non-adhesive end edge is preferably also folded back (such as along F₃ and F₄) to provide a graspable tab 116 and 118 for the consumer to hold in order to more easily remove the release papers 112 and 114.

In other embodiments, the end edges of the release papers may abut, rather than overlap. In still other (but, less preferred) embodiments, the end edges may be spaced slightly apart. Numerous other embodiments of multiple release paper arrangements are also possible. For example, in other embodiments, the multiple release papers may be arranged in a side-by-side arrangement, rather than end to end. In these or other embodiments, more than two release papers can be used. The multiple release paper embodiments described above are particularly useful when the main body portion 22 of the sanitary napkin is extensible, highly flexible, or both. Such multiple release paper arrangements provide ease in handling these types of sanitary napkins and allow the wearer to place the same in her panties without portions of the adhesive fastener on the sanitary napkin folding over and inadvertently sticking to other portions of the sanitary napkin.

In the multiple piece release paper embodiments, or in any of the other embodiments described herein, the release paper could be provided with additional stiffness to aid in handling extensible and/or highly flexible sanitary napkins. This release paper is preferably stiffer than release papers currently in use. This will allow the sanitary napkin 20 to have stiffness when desired, such as to aid in handling the sanitary napkin, without altering the properties of the sanitary napkin when flexibility is desired, such as when the sanitary napkin 20 is placed in the wearer's panties.

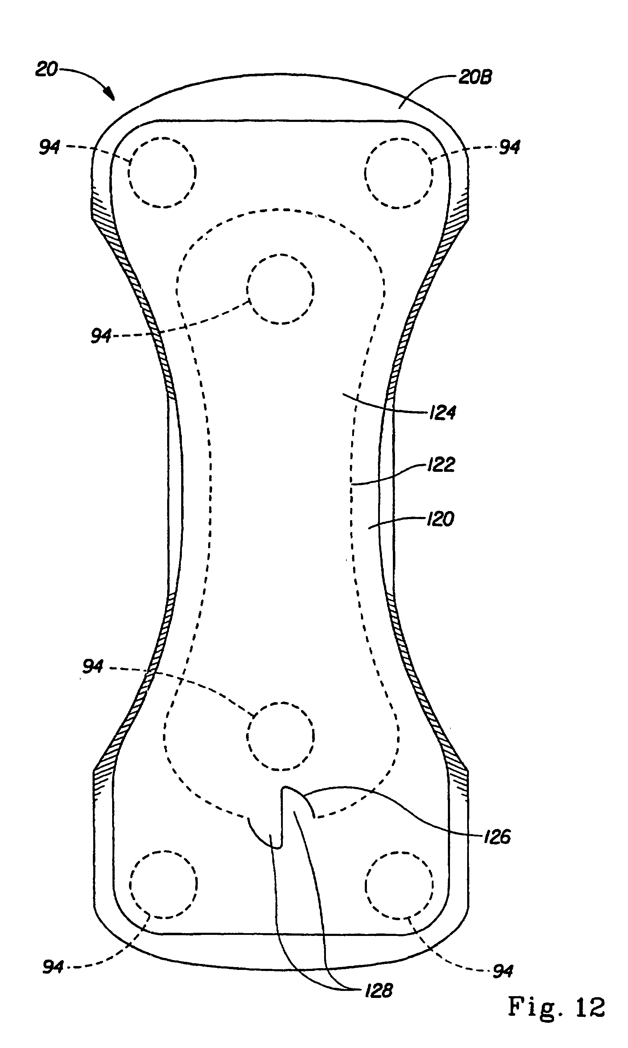

FIG. 12 shows another alternative way of covering the adhesive fasteners 94 on the garment facing side of the sanitary napkin. In the embodiment shown in FIG. 12, the adhesive fasteners 94 are disposed in the configuration of a number of spaced apart circular regions of adhesive. (It should be understood, however, that this adhesive fastener configuration is merely for purposes of illustration, and that this adhesive cover embodiment, like the previous embodiments, is not limited to use with the particular adhesive fastener configuration shown in the drawings.) The adhesive fasteners 94 are covered with a release paper 120 that is perforated so that it can be separated into more than one piece for removal. More specifically, the embodiment shown in FIG. 12 comprises an hourglass-shaped release strip 120 that has a perforated line 122 in a dog bone shape in the center. The perforated line 122 allows the user to separately remove the dog bone-shaped piece 124 and the remainder of the release paper 120. The release paper 120 and the perforation line 122, however, can be in any desired configuration that allows the release paper 120 to be separated into more than one piece for removal. The embodiment shown in FIG. 12 provides the advantage that it allows the wearer to remove selected portions of the release strip so that certain portions of the adhesive fastener 94 will be exposed to control the amount and location of the adherence of the sanitary napkin 20 to their undergarment.

In a particularly preferred embodiment, one or more of the pieces of the release paper 120 can be provided with a graspable portion to assist the user in removing the separate pieces of release paper. This graspable portion or portions can be formed in any manner, and can be in any configuration that is suitable for this purpose. In the embodiment shown in FIG. 12, this as accomplished by providing a continuous cut 126 that is connected to the perforated line 122. The continuous cut 126 is generally "S"-shaped so that it forms a graspable tab 128 for each of the pieces of release paper to more easily remove the same.

Numerous other alternative embodiments of the absorbent article and wrappers described herein are possible. For example, suitable absorbent articles in the form of pantiliners that could be provided with the features of the present invention are disclosed in U.S. Patent 4,738,676 entitled "Pantiliner" issued to Osborn on April 19, 1988. Suitable absorbent articles, at least some of which are in the form of adult incontinence products that could be provided with the features of the present invention, are described in U.S. Patents 5,300,054 issued to Feist, et al. on April 5, 1994, and 5,304,161 issued to Noel, et al. on April 19, 1994. In still other embodiments, the features of the present invention could be provided on an article that is in the form of an undergarment shield which may have limited absorbency, and the shield could be placed in the wearer's undergarments with an absorbent pad positioned on top of the shield.

The disclosures of all patents, patent applications (and any patents which issue thereon, as well as any corresponding published foreign patent applications), and publications mentioned throughout this description are hereby incorporated by reference herein. It is expressly not admitted, however, that any of the documents incorporated by reference herein teach or disclose the present invention.

## Claims

1. An absorbent article (20) for wearing in a wearer's undergarment that has a crotch region with a pair of side edges, said absorbent article (20) having a principal longitudinal centerline L, principal transverse centerline T, a longitudinal dimension extending in a longitudinal direction, and a transverse dimension extending in a transverse direction, said absorbent article (20) comprising:
a main body portion (22) having a body-facing side, a garment-facing side, a pair of end regions (32,34), a central region (36) disposed between said end regions (32,34), said central region (36) preferably being thicker than said end regions (32,34), and two spaced apart longitudinal side edges (26), a portion of which comprise the laterally outwardmost portion of said main body portion (22), and two spaced apart end edges (28), said main body portion (22) comprising a liquid pervious topsheet (44), a liquid impervious backsheet (46) joined to said topsheet (44), and an absorbent core (48) positioned between said topsheet (44) and said backsheet (46), said main body portion (22) being narrower in width at said central region (36) than at said end regions (32,34); and
a pair of side wrapping elements (24) for folding around the side edges of the wearer's undergarment, said side wrapping elements (24) having distal edges (76) and being joined to said main body portion (22) and extending laterally outward to said distal edges (76) from at least said central region (36) of said main body portion (22), said side wrapping elements (24) being sufficiently flexible and sized and configured so that they are capable of folding around the side edges of a crotch region of a wearer's undergarment, said side wrapping elements (24) having a surface area wherein the majority of said surface area of said side wrapping elements (24) is located laterally inward of the laterally outwardmost portion of said main body portion (22), **characterised in that** the distal edges (76) of said side wrapping elements (24) are generally straight.

2. The absorbent article (20) of Claim 1 wherein said longitudinal side edges (26) of said main body portion (22) in said central region (36) are concave relative to the distal edges (76) of said side wrapping elements (24).

3. The absorbent article (20) of Claim 2 wherein said side wrapping elements (24) are more flexible than said main body portion (22), and said absorbent article (20) is provided with a curved hinge line for said side wrapping elements (24) to fold about that is located between at least a portion of said main body portion (22) and the distal edges (76) of said side wrapping elements (24).

4. The absorbent article (20) of Claim 3 wherein said side wrapping elements (24) are provided with at least one region (84) that is more flexible, and preferably also more extensible, than the adjacent regions (86) of said side wrapping elements (24), said more flexible and extensible region (84) preferably comprising a corrugated portion of said side wrapping element (24).

5. The absorbent article (20) of Claim 4 wherein said side wrapping elements (24) have a transverse centerline T_{1,} and said side wrapping elements (24) comprise a pair of flexible regions (84) positioned adjacent said longitudinal side edges (26) of said main body portion (22), and being disposed on opposite sides of said transverse centerline T₂ of said side wrapping elements (24).

6. The absorbent article (20) of Claim 2 wherein said side wrapping elements (24) have a curvilinear folding axis adjacent said longitudinal side edge (26) of said main body portion (22), and wherein said side wrapping elements (24) preferably have a garment-facing side with fasteners (96) thereon allowing said side wrapping elements (24) to be folded under the crotch region of a wearer's undergarments and attached to the underside of the crotch of said undergarment.

7. The absorbent article (20) of Claim 1 wherein said main body portion (22) comprises a primary absorbent component (50) comprising airfelt, and a secondary absorbent component (52) comprising a thermally bonded airlaid nonwoven material comprising a blend of cellulose and bicomponent fibers.

8. The absorbent article (20) of Claim 1 wherein said side wrapping elements (24) are capable of covering greater than or equal to 160 mm, preferably greater than or equal to 170 mm, and more preferably greater than or equal to 200 mm, of the side edges of a wearer's undergarments.

9. A method of making an absorbent article (20) according to claim 1, such as a sanitary napkin, having side wrapping elements (24), **characterized in that** said method comprises the steps of:
(a) providing a web of topsheet material (44) for said sanitary napkin (20), said web of topsheet material having generally rectilinear side edges;
(b) providing a web of backsheet material (46) for said sanitary napkin (20), said web of backsheet material having generally rectilinear side edges;
(c) providing a web of absorbent material (52) for said sanitary napkin (20), said web of absorbent material having generally rectilinear side edges;
(d) forming cuts (90) in said absorbent material along each side edge of said absorbent material, said cuts (90) in said absorbent material (52) being disposed about a transverse centerline and being generally curvilinear and concave and extending inward from the side edges of said absorbent material (52) toward, but not all the way to the transverse centerline to form a curvilinear side edge (93) in said absorbent material (52) and outer portions (92) of said absorbent material (52) that lie laterally outboard of said cut (90), wherein said outer portions (92) have a generally curvilinear proximal edges and a generally rectilinear distal edge;
(e) folding said outer portions (92) of said absorbent material (52) longitudinally inward toward said longitudinal centerline of said web of absorbent material (52);
(f) placing said topsheet (44) on one side of said absorbent material (52) and said backsheet (46) on the opposite side, wherein said topsheet (44) and said backsheet (46) extend laterally outward beyond the curvilinear side edge (93) formed in said absorbent material (52) to form side wrapping elements (24); and
(g) joining at least a portion of the periphery of said topsheet (44) to said backsheet (46).

## Patentansprüche

1. Absorbierender Artikel (20) zum Tragen in einer Unterwäsche eines Trägers, die eine Schrittregion mit ein Paar Seitenrändern hat, wobei der absorbierende Artikel (20) eine längs verlaufende Haupt-Mittellinie L, eine quer verlaufende Haupt-Mittellinie T, eine sich in einer Längsrichtung erstreckende Längsabmessung und eine sich in einer Querrichtung erstreckende Querabmessung aufweist, wobei der absorbierende Artikel (20) umfaßt:
einen Hauptkörperbereich (22) mit einer körperseitigen Seite, einer wäscheseitigen Seite, einem Paar Endregionen (32, 34), einer zentralen Region (36), die zwischen den Endregionen (32, 34) angeordnet ist, wobei die zentrale Region (36) vorzugsweise dicker ist als die Endregionen (32, 34), und zwei in Abstand zueinander liegenden längs verlaufenden Seitenrändern (26), von denen ein Abschnitt den seitlich äußersten Bereich des Haupt-Körperbereichs (22) umfaßt, und zwei in Abstand zueinander liegenden Stirnrändern (28), wobei der Hauptkörperbereich (22) eine flüssigkeitsdurchlässige Oberschicht (44) eine mit der Oberschicht (44) verbundene flüssigkeitsundurchlässige Unterschicht (46) und einen zwischen der Oberschicht (44) und der Unterschicht (46) positionierten, absorbierenden Kern (48) umfaßt, wobei der Hauptkörperbereich (22) an der zentralen Region (36) in seiner Breite schmaler ist als an den Endregionen (32, 34); und
ein Paar Seiten-Hüllelemente (24) zum Herumfalten um die Seitenränder der Unterwäsche des Trägers, wobei die Seiten-Hüllelemente (24) distale Ränder (76) haben und mit dem Hauptkörperbereich (22) verbunden sind und sich zu den distalen Rändern (76) von wenigstens der zentralen Region (36) des Hauptkörperbereichs (22) aus seitlich nach außen erstrecken, wobei die Seiten-Hüllelemente (24) ausreichend flexibel und bemessen und konfiguriert sind, so daß sie in der Lage sind, sich um die Seitenränder der Schrittregion der Unterwäsche eines Trägers herum zu falten, wobei die Seiten-Hüllelemente (24) einen Oberflächenbereich haben, in welchem der Hauptteil des Oberflächenbereichs der Seiten-Hüllelemente (24) seitlich innerhalb des seitliche äußersten Bereichs des Hauptkörperbereichs (22) liegt, **dadurch gekennzeichnet, daß** die distalen Ränder (76) der Seiten-Hüllelemente (24) im wesentlichen gerade sind.

2. Absorbierender Artikel (20) nach Anspruch 1, in welchem die Längsseitenränder (26) des Hauptkörperbereichs (22) in der zentralen Region (36) in Bezug zu den distalen Rändern (76) der Seiten-Hüllelemente (24) konkav sind.

3. Absorbierender Artikel (20) nach Anspruch 1, in welchem die Seiten-Hüllelemente (24) flexibler sind als der Hauptkörperbereich (22) und der absorbierende Artikel (20) mit einer gekrümmten Gelenklinie versehen ist, um die sich die Seiten-Hüllelemente (24) falten lassen, die zwischen wenigstens einem Abschnitt des Hauptkörperbereichs (22) und den distalen Rändern (76) der Seiten-Hüllelemente (24) angeordnet ist.

4. Absorbierender Artikel (20) nach Anspruch 3, in welchem die Seiten-Hüllelemente (24) mit wenigstens einer Region (84) versehen sind, die flexibler und vorzugsweise auch streckfähiger sind, als die benachbarten Regionen (86) der Seiten-Hüllelemente (24), wobei die flexible und streckfähige Region (84) vorzugsweise einen geriffelten Bereich der Seiten-Hüllelemente (24) umfaßt.

5. Absorbierender Artikel (20) nach Anspruch 4, in welchem die Seiten-Hüllelemente (24) eine quer verlaufende Mittellinie T 1 haben, und wobei die Seiten-Hüllelemente (24) ein Paar flexible Regionen (84) umfassen, die angrenzend an die Längsseitenränder (26) des Hauptkörperbereichs (22) positioniert sind und auf gegenüber liegenden Seiten der quer verlaufenden Mittellinie T1 der Seiten-Hüllelemente (24) angeordnet sind.

6. Absorbierender Artikel (20) nach Anspruch 2, in welchem die Seiten-Hüllelemente (24) eine kurvenlineare Faltachse angrenzend an den Längsseitenrand (26) des Hauptkörperbereichs (22) haben, und in welchem die Seiten-Hüllelemente (24) vorzugsweise eine wäscheseitige Seite mit Befestigern (96) darauf haben, welche den Seiten-Hüllelementen (24) erlauben, unter die Schrittregion der Unterwäsche eines Trägers gefaltet zu werden und an der Unterseite des Schritts der Unterwäsche angebracht zu werden.

7. Absorbierender Artikel (20) nach Anspruch 1, in welchem der Hauptkörperbereich (22) eine primäre absorbierende Komponente (50) mit Luftfilz und eine sekundäre absorbierende Komponente (52) mit einem thermisch gebundenen, luftgelegten Vliesmaterial mit einem Gemisch aus Zellulose und Bikomponentfasern umfaßt.

8. Absorbierender Artikel (20) nach Anspruch 1, in welchem die Seiten-Hüllelelemente (24) in der Lage sind mehr als oder gleich 160 mm, vorzugsweise mehr als oder gleich 170 mm und ganz bevorzugt mehr als oder gleich 200 mm der Seitenränder der Unterwäsche eines Trägers zu überdecken.

9. Verfahren zum Herstellen eines absorbierenden Artikels (20) gemäß Anspruch 1, wie eine Damenbinde, mit Seiten-Hüllelementen (24), **dadurch gekennzeichnet, daß** das Verfahren die Schritte umfaßt:
(a) Bereitstellen einer Bahn eines Oberschichtmaterials (44) für die Damenbinde (20), wobei die Bahn des Oberschichtmaterials im wesentlichen geradlinige Seitenränder hat;
(b) Bereitstellen einer Bahn eines Unterschichtmaterials (46) für die Damenbinde (20), wobei die Bahn aus Unterschichtmaterial im wesentlichen geradlinige Seitenränder hat;
(c) Bereitstellen einer Bahn eines absorbierenden Materials (52) für die Damenbinde (20), wobei die Bahn aus absorbierendem Material im wesentlichen geradlinige Seitenränder hat;
(d) Bilden von Schnitten (90) in dem absorbierenden Material entlang jedes Seitenrandes des absorbierenden Materials, wobei die Schnitte (90) in dem absorbierenden Material (52) um eine quer verlaufende Mittellinie herum angeordnet sind und im wesentlichen kurvenlinear und konkav sind und sich von den Seitenrändern des absorbierenden Materials (52) nach innen zu aber nicht über den ganzen Weg zu der quer verlaufenden Mittellinie hin erstrecken, um einen kurvenlinearen Seitenrand (93) in dem absorbierenden Material (52) und äußere Bereiche (92) des absorbierenden Materials (52), die seitlich außerhalb des Schnittes (90) liegen, zu bilden, wobei die äußeren Bereiche (92) im wesentlichen kurvenlineare proximale Ränder und einen im wesentlichen geraden distalen Rand haben;
(e) Falten der äußeren Bereiche (92) des absorbierenden Materials (52) in Längsrichtung nach innen, in Richtung auf die längs verlaufende Mittellinie der Bahn des absorbierenden Materials (52);
(f) Anordnen der Oberschicht (44) auf einer Seite des absorbierenden Materials (52) und der Unterschicht (46) auf der entgegen gesetzten Seite, wobei sich die Oberschicht (44) und die Unterschicht (46) seitlich über den kurvenlinearen Seitenrand (93), der in dem absorbierenden Material (52) ausgebildet ist, nach außen erstrekken, um Seiten-Hüllelemente (24) zu bilden; und
(g) Verbinden wenigstens eines Bereichs des Umfanges der Oberschicht (44) mit der Unterschicht (46).

## Revendications

1. Article absorbant (20) destiné à être porté dans le sous-vêtement d'un utilisateur, dont la région d'entrejambe comporte une paire de bords latéraux, ledit article absorbant (20) présentant une ligne médiane principale longitudinale L, une ligne médiane principale transversale T, une dimension longitudinale s'étendant dans une direction longitudinale et une dimension transversale s'étendant dans une direction transversale, ledit article absorbant (20) comprenant :
une partie de corps principal (22) comportant un côté faisant face au corps, un côté faisant face au vêtement, une paire de régions d'extrémité (32, 34), une région centrale (36) placée entre lesdites régions d'extrémité (32, 34), ladite région centrale (36) étant, de préférence, plus épaisse que lesdites régions d'extrémité (32, 34), et deux bords de côté longitudinal (26) espacés, dont une partie comprend la partie latéralement la plus extérieure de ladite partie de corps principal (22), et deux bords d'extrémité (28) espacés, ladite partie de corps principal (22) comprenant une feuille de dessus (44) perméable aux liquides, une feuille de fond (46) imperméable aux liquides réunie à ladite feuille de dessus (44), et une âme absorbante (48) placée entre ladite feuille de dessus (44) et ladite feuille de fond (46), ladite partie de corps principal (22) ayant une largeur plus étroite au niveau de ladite région centrale ((36) qu'au niveau desdites régions d'extrémité (32, 34); et
une paire d'éléments enveloppants latéraux (24) destinés à se plier autour des bords latéraux du sous-vêtement de l'utilisateur, lesdits éléments enveloppants latéraux (24) présentant des bords distaux (76) et étant réunis à ladite partie de corps principal (22) et s'étendant latéralement vers l'extérieur vers lesdits bords distaux (76) au moins à partir de ladite région centrale (36) de ladite partie de corps principal (22), lesdits éléments enveloppants latéraux (24) étant suffisamment flexibles et dimensionnés et configurés afin qu'ils soient capables de se plier autour des bords latéraux de la région d'entrejambe du sous-vêtement de l'utilisateur, lesdits éléments enveloppants latéraux (24) présentant une certaine superficie,
dans lequel la plus grande partie de ladite superficie desdits éléments enveloppants latéraux (24) est située latéralement vers l'intérieur de la partie latéralement la plus extérieure de ladite partie de corps principal (22),
**caractérisé en ce que** les bords distaux (76) desdits éléments enveloppants latéraux (26) sont globalement droits.

2. Article absorbant (20) selon la revendication 1, dans lequel lesdits bords de côté longitudinal (26) de ladite partie de corps principal (22), dans ladite région centrale (36), sont concaves par rapport aux bords distaux (76) desdits éléments enveloppants latéraux (24).

3. Article absorbant (20) selon la revendication 2, dans lequel lesdits éléments enveloppants latéraux (24) sont plus flexibles que ladite partie de corps principal (22), et ledit article absorbant (20) est prévu avec une ligne d'articulation incurvée pour lesdits éléments enveloppants latéraux (24) afin qu'ils se plient autour de celle-ci, qui est située au moins entre une partie de ladite partie de corps principal (22) et les bords distaux (76) desdits éléments enveloppants latéraux (24).

4. Article absorbant (20) selon la revendication 3, dans lequel lesdits éléments enveloppants latéraux (24) sont prévus au moins avec une région (84) qui est plus flexible et aussi, de préférence, plus extensible que les régions adjacentes (86) desdits éléments enveloppants latéraux (24), ladite région plus flexible et extensible (84) comprenant, de préférence, une région ondulée desdits éléments enveloppants latéraux (24).

5. Article absorbant (20) selon la revendication 4, dans lequel lesdits éléments enveloppants latéraux (24) présentent une ligne médiane transversale T1, et lesdits éléments enveloppants latéraux (24) comprennent une paire de régions flexibles (84) placées à proximité immédiate desdits bords de côté longitudinal (26) de ladite partie de corps principal (22), et placées sur les côtés opposés de ladite ligne médiane transversale T1 desdits éléments enveloppants latéraux (24).

6. Article absorbant (20) selon la revendication 2, dans lequel lesdits éléments enveloppants latéraux (24) présentent un axe de pliage curviligne à proximité immédiate dudit bord de côté longitudinal (26) de ladite partie de corps principal (22), et dans lequel lesdits éléments enveloppants latéraux (24) présentent, de préférence, un côté faisant face au vêtement sur lequel se trouvent des dispositifs d'attache (96) permettant que lesdits éléments enveloppants latéraux (24) soient pliés sous la région d'entrejambe du sous-vêtement de l'utilisateur et fixés au côté inférieur de l'entrejambe dudit sous-vêtement.

7. Article absorbant (20) selon la revendication 1, dans lequel ladite partie de corps principal (22) comprend un composant absorbant primaire (50) comprenant un feutre aéré, et un composant absorbant secondaire (52) comprenant un matériau non tissé appliqué par jet d'air, thermorelié comprenant un mélange de fibres de cellulose et bicomposants.

8. Article absorbant (20) selon la revendication 1, dans lequel lesdits éléments enveloppants latéraux (24) sont capables de recouvrir une longueur supérieure ou égale à 160 mm, de préférence, supérieure ou égale à 170 mm et, mieux encore, supérieure ou égale à 200 mm des bords latéraux du sous-vêtement de l'utilisateur.

9. Procédé de fabrication d'un article absorbant (20) selon la revendication 1, tel qu'une serviette hygiénique, comportant des éléments enveloppants latéraux (24), **caractérisé en ce que** ledit procédé comprend les étapes consistant à :
(a) fournir une nappe de matériau de feuille de dessus (44) pour ladite serviette hygiénique (20), ladite nappe de matériau de feuille de dessus présentant des bords latéraux généralement rectilignes;
(b) fournir une nappe de matériau de feuille de fond (46) pour ladite serviette hygiénique (20), ladite nappe de matériau de feuille de fond présentant des bords latéraux généralement rectilignes ;
(c) fournir une nappe de matériau absorbant (52) pour ladite serviette hygiénique (20), ladite nappe de matériau absorbant présentant des bords latéraux généralement rectilignes;
(d) former des découpes (90) dans ledit matériau absorbant le long de chaque bord latéral dudit matériau absorbant, lesdites découpes (90) ménagées dans ledit matériau absorbant (52) étant placées autour d'une ligne médiane transversale et étant généralement curvilignes et concaves et s'étendant vers l'intérieur à partir des bords latéraux dudit matériau absorbant (52) dans la direction mais pas dans toute la direction de la ligne médiane transversale afin de former un bord latéral curviligne (93) dans ledit matériau absorbant (52) et les parties extérieures (92) dudit matériau absorbant (52) qui se trouvent latéralement vers l'extérieur de ladite découpe (90), étape dans laquelle lesdites parties extérieures (92) présentent des bords proximaux généralement curvilignes et un bord distal généralement rectiligne;
(e) plier lesdites parties extérieures (92) dudit matériau absorbant (52) longitudinalement vers l'intérieur en direction de ladite ligne médiane longitudinale de ladite nappe du matériau absorbant (52) ;
(f) mettre en place ladite feuille de dessus (44) sur un côté dudit matériau absorbant (52) et ladite feuille de fond (46) sur le côté opposé, étape dans laquelle ladite feuille de dessus (44) et ladite feuille de fond (46) s'étendent latéralement vers l'extérieur au-delà du bord latéral curviligne (93) formé dans ledit matériau absorbant (52) afin de former les éléments enveloppants latéraux (24); et
(g) réunir au moins une partie de la périphérie de ladite feuille de dessus (44) à ladite feuille de fond (46).
